# EUROPEAN PATENT SPECIFICATION

(11) **EP 1 029 527 B1**
(45) Date of publication and mention of the grant of the patent: **28.02.2007**
(21) Application number: 98936677.8
(22) Date of filing: 06.08.1998
(51) Int. Cl.: A61K 8/70

(54) **COSMETIC PREPARATION**
KOSMETISCHE ZUSAMMENSETZUNG
PREPARATION COSMETIQUE

(30) Priority: 28.08.1997 JP 23289197
(43) Date of publication of application: 23.08.2000
(73) Proprietor: DAIKIN INDUSTRIES, LIMITED, Osaka-shi, Osaka 530-0015 (JP)
(72) Inventor: MORITA, Masamichi Yodogawa Works, Settsu-shi Osaka 566-0044 (JP); KUBO, Motonobu Yodogawa Works, Settsu-shi, Osaka 566-0044 (JP)
(74) Representative: HOFFMANN EITLE
(86) International application number: PCT/JP1998/003499
(87) International publication number: WO 1999/011225

(56) References cited:
- EP-A- 1 000 602
- WO-A1-96/22356
- JP-A- 6 224 913
- JP-A- 6 227 942
- JP-A- 7 133 210
- JP-A- 62 123 107
- JP-A- 62 223 105
- JP-A- 62 249 913
- US-A- 4 803 067
- US-A- 5 304 334
- US-A- 5 330 681
- US-A- 5 571 858
- US-A- 5 578 311
- US-A- 5 667 772
- US-A- 5 851 539
- US-A- 5 925 611
- US-A- 6 002 048
- US-A- 6 060 626
- PATENT ABSTRACTS OF JAPAN vol. 012, no. 186 (C-500), 31 May 1988 (1988-05-31) & JP 62 292713 A (KANEBO LTD), 19 December 1987 (1987-12-19)
- PATENT ABSTRACTS OF JAPAN vol. 012, no. 198 (C-502), 8 June 1988 (1988-06-08) & JP 63 002916 A (KANEBO LTD), 7 January 1988 (1988-01-07)
- HFE-7100 Product Information Sheet, June 1996
- OWENS J. ET AL.: "Performance of Hydrofluoroethers in Cleaning Applications" INTERNATIONAL CFC & HALON ALTERNATIVES CONFERENCE, November 1995 (1995-11), pages 1-8, WASHINGTON D.C.
- PANTINI G. ET AL: "Perfluoropolyethers Status and New Developments" COSMETICS & TOILETRIES, vol. 106, October 1991 (1991-10), pages 71-80,
- SEKIYA A. ET AL.: "A Continuing Search for New Refrigerants" CHEMTECH, December 1996 (1996-12), pages 44-48,
- 3M Website, 22 May 1997

## Description

### FIELD OF THE INVENTION

The present invention relates to a cosmetic comprising hydrofluoroether (abbreviated hereinafter into "HFE") having a viscosity of less than 5 mPa·s at 25°C which is a fluorine-containing solvent causing no environmental disruption and being highly safe for the skin. The HFE is characterized by these properties as well as by high solubility in fluorine-containing oil having a viscosity of at least 5 mPa·s at 25°C and high dispersibility in a fluorine compound-treated powder.

### RELATED ART

In recent years, cosmetics incorporating volatile solvents such as isoparaffin and cyclic silicone are frequently used. These volatile solvents have the advantage that upon application there is a refreshing feeling and their solubility is high, and their use can easily improve the feeling of cosmetics in use and increase the functionality thereof. However, isoparaffin and cyclic silicone severely irritate the skin and have the problem of their adverse influence on the environment after volatilization.

Fluorocarbons such as perfluorohexane, chlorofluorocarbon (CFC) and hydrochlorofluorocarbon (HCFC) have also been examined as substitutes for these solvents. Because the ozone depletion coefficient and warming coefficient of these solvents are high, there is the disadvantage that they are highly dangerous owing to environmental disruption.

3M Published PCT Application No. WO 96/22356 refers to a cleaning process and composition. Both the cleaning process and the composition are supposed to be used to remove contaminants from the surface of a substrate. The cleaning composition comprises at least one mono-, di-, or trialkoxy-substituted perfluoroalkane, perfluorocycloalkane, perfluorocyclo-alkyl-containing perfluoroalkane, or perfluorocycloalkylene-containing perfluoroalkane compound. It becomes clear from WO 96/22356 (Flynn et al. PCT) that the cleaning process and composition is supposed to be used in solvent cleaning applications. This document does not relate to any cosmetic-related applications.

The HFE-7100 product information sheet of June 1996 describes HFE-7100, which is a hydrofluoroether of 3M. HFE-7100 is a methoxy-nonafluorobutane (C₄F₉OCH₃), and is described as a clear, colorless and low-odor fluid intended to replace ozone-depleting materials.

Again, clearly the applications mentioned for the hydrofluoroether HFE-7100 do not come under the art of cosmetics but a completely different field of the art.

Owens et al., "Performance of Hydrofluoroethers in Cleaning Applications" (Oct. 95) describes hydrofluoroethers (HFEs) as a promising new class of alternative solvents and is not concerned with cosmetics at all.

In U.S. Patent No. 4, 803,067, it was an object to solve the problem of difficulties with emulsions consisting of the choice of an emulsifier effective for the perfluorinated compounds, and obtaining sufficiently stable products. There is no indication in U.S. Patent No. 4, 803,067 that specific hydrofluoroethers with a specific viscosity can be used as dissolving or dispersion agents to obtain improved results according to the present invention.

From U.S. Patent No. 5, 304, 334 methods of preparing and stabilizing complex multiphase compositions are known that include distinct phases of water, gel, liquid crystal or oil and perfluoropolyether dispersed in a continuous phase of a silicone fluid.

The dispersion agent according to U.S. 5, 304, 334 is thereby silicone fluid and is not a dispersion or dissolving agent being a hydrofluoropolyether.

U.S. Patent No. 5,330 681 relates to stable emulsions of perfluoropolyethers in particular in the field of cosmetic and dermatological specialties. The stable diphase emulsions of document U.S. 5,330 681 consist of
- perfluoropolyethers having perfluoroalkyl end groups
- conventional surfactants, dispersed in
- a continuous dispersing phase consisting of glycerol or of a polyhyxdroxylated compound containing at least three hydroxyl groups, selected from polyalcohols and saccharides, dissolved in a hydrophilic solvent and/or water.

U.S. Patent No. 5,571,858 refers specifically to aqueous nail varnishes which can contain 10 to 59% by weight of a film forming polymer present in a dispersed state, and from 0.01 to 1% by weight of a water-soluble perfluoroalkyl compound in addition to 40 to 90% by weight of water. It is stated that the presence of the water-soluble perfluoroalkyl compound makes it possible to provide excellent aqueous nail varnishes with improved spreading properties. Said document does not make mention of specific hydrofluoroethers.

From U.S. Patent No. 5,578,311 a cosmetic is known which comprises at least a fluorine compound treated powder and a liquid perfluoro organic compound, wherein the fluorine compound treated powder amounts from 5 to 95% by weight and wherein the liquid perfluoro organic compounds amount from 5 to 95% by weight. The fluorine compound treated powder preferably has the following general formula (I):

(CₛF₂ₛ₊₁C₁H₂₁O)₁PO(OM)₃₋₁

To use fluorohydrocarbon as a blending solvent was known from Japanese Publ. Patent Appln. No. 62-123107, wherein the fluorohydrocarbon is blended with a powdery cosmetic base. It is stated that the fluorohydrocarbon has a good affinity for the cosmetic base and that stirring and blending are facilitated.

Japanese Published Patent Application No. 6-227942 relates to a cleansing agent composition containing a fluorine compound in addition to a liquid oily base. In a preferred embodiment, the cleansing agent composition further contains a surfactant.

Pantini et al., "Perfluoropolyethers Status and New Developments," Cosmetics & Toiletries, pp. 71-80, vol. 106, (Oct. 1991) is an article from the international magazine of product development in cosmetics and toiletries. It refers to perfluoropolyethers which can be used in cremes and lotions, shampoos and bath products, soaps and syndets, lipsticks and other makeup preparations. It is stated that perfluoropolyethers have the ability to form a protective film against a wide range of aggressive chemicals. The perfluoropolyethers in this article are prepared preferably in glycerine emulsions.

Sekiya et al., "A continuing search for new refrigerants," Chemtech, pp. 44-48 (Dec. 1996) is an article from the Magazine of Innovation in Chemistry and Technology. Reference is made in the article to the continuing search for new refrigerants. It is stated that the destruction of the ozone layer by chlorofluorocarbons is the first environmental issue to be considered on a global scale. Hydrofluorocarbons are recited as an alternative. There is no indication of utility of such compounds in cosmetic applications.

Japanese Published Patent Application No. 62-223105 refers to a multi-phase emulsion cosmetic which is obtained by dissolving a specific fluorine-containing surfactant in an oil component, dispersing and emulsifying a perfluoro-organic compound in the solution to obtain an emulsion and dispersing and emulsifying the emulsion in an aqueous solution of a water-based emulsifier.

Japanese Published Patent Application No. 62-249913 refers to a cosmetic which is obtained by dissolving a specific lipophilic fluorine surface active agent in a perfluoro organic compound, dispersing and emulsifying an oil component in the solution and dispersing and emulsifying the emulsion in an aqueous solution of a specific hydrophilic surface active agent.

The 3M Website, May 22, 1997 relates to precision and electronics cleaning. There again, the 3M HFEs (hydrofluoroethers) are described which are considered as offering a performance which was already known from CFC and other ozone-depleting substances, without the risks and compromises of typical ODS replacements. Thereby, the 3M Website relates to industrial cleaning applications.

### SUMMARY OF THE INVENTION

As a result of the intense study for solving the problem described above, the present inventors found that a partially fluorinated fluorine-containing solvent, the HFE, is highly safe for the skin and has less or no influence on the environment. In addition to the properties described above, the HFE has high solubility in a fluorine-containing oil used frequently in recent cosmetics and high dispersibility in fluorine compound-treated powders, thus easily permitting improvements in the feeling of cosmetics upon use, and giving a high functionality.

The present invention provides a cosmetic comprising at least 1% by weight of the HFE having a viscosity of less than 5 mPa·s at 25°C, represented by the general formula (1):
A cosmetic comprising at least 1% by weight of a hydrofluoroether having a viscosity of less than 5 mPa·s at 25°C, represented by the general formula (1):

   CₙF₂ₙ₊₁-O-Cₓ H₂ₓ₊₁ (1)
wherein n is a number of 1 to 6, and x is a number of 1 to 6, with the proviso (for FR, GB, IT) that the cosmetic does not comprise a copolymer comprising
(A) 5 to 95% by weight of repeating units derived from a fluorine-containing (meth)acrylate, and
(B) 95 to 5 % by weight of repeating units derived from at least one fluorine-free monomer selected from the group consisting of a silicone macromonomer, a polyalkylene glycol (meth)acrylate, an alkyl (meth)acrylate macromonomer, and an alkyl (meth)acrylate,
wherein the copolymer comprises:
(i) a fluorine-containing (meth)acrylate and a silicone macromonomer,
(ii) a fluorine-containing (meth)acrylate and a polyalkylene glycol (meth)acrylate,
(iii) a fluorine-containing (meth)acrylate and an alkyl (meth)acrylate macromonomer,
(iv) a fluorine-containing (meth)acrylate and a C₁₋₄ alkyl-containing alkyl (meth)acrylate,
(v) a fluorine-containing (meth)acrylate, a silicone macromonomer, and a C₁₋₂₂ alkyl-containing alkyl (meth)acrylate, or
(vi) a fluorine-containing (meth)acrylate, a silicone macromonomer, and an alkyl (meth)acrylate macromonomer.

The cosmetic of the present invention serve as a finishing cosmetic such as foundation, face powder, cheek color and eye color, a fundamental cosmetic such as face lotion, milky lotion, cream and sunscreen milky lotion, and hair-caring products such as rinse and conditioner, and lipstick overcoat.

### DETAILED DESCRIPTION OF THE INVENTION

In the formula (1) n is 1 to 6, and x is 1 to 6.

Specific examples of the HFE include:
C₄F₉OCH₃, C₄F₉OC₂H₅, C₄F₉OC₃H₇, C₅F₁₁OC₂H₅, C₃F₇OC₄H₉ and C₄F₉OC₄H₉.

The HFE may be incorporated alone into the cosmetic or may be used as a mixture of at least two types. The amount of the HFE is at least 1% by weight, for example 1 to 99.9% by weight, preferably 5 to 99% by weight, particularly 10 to 99% by weight, based on the cosmetic. The cosmetic comprising the HFE can be produced in a conventional manner.

The HFE is advantageously superior in the ability to dissolve a fluorine-containing oil having a viscosity of at least 5 mPa·s at 25°C. By virtue of this advantage, the cosmetic having the advantage never achieved can be produced. It is preferable that at least 1% by weight of the fluorine-containing oil and at least 5% by weight of the HFE are contained in the cosmetic. For example, the amount of the fluorine-containing oil is preferably 1 to 90% by weight, particularly 5 to 50% by weight, for example 5 to 20% by weight, based on the cosmetic. For example, a weight ratio of the fluorine-containing oil to the HFE may be in the range of from 1:100 to 1:5, preferably from 1:90 to 1:10.

The fluorine-containing oil having a viscosity of at least 5 mPa·s may be, for example, a perfluoropolyether, or a fluorine-based oil represented by the general formula (2): 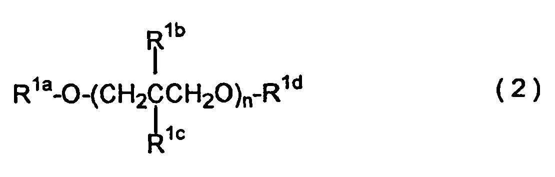 wherein R^{1a} and R^{1d} are each a hydrogen atom or a partially or completely fluorinated aliphatic group having 1 to 20 carbon atoms; R^{1b} and R^{1c} are each a hydrogen atom, or an aliphatic group having 1 to 20 carbon atoms, or a partially or completely fluorinated aliphatic group having 1 to 20 carbon atoms, with the proviso that at least one of R^{1a} to R^{1d} is a partially or completely fluorinated aliphatic group having 1 to 20 carbon atoms; and n is a number of 1 to 20, or a fluorine-based oil represented by the general formula (3): 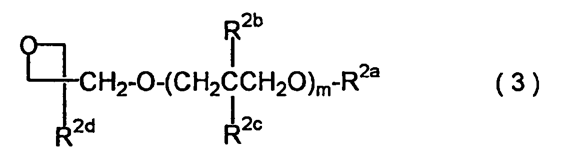 wherein R^{2a} is a hydrogen atom or a partially or completely fluorinated aliphatic group having 1 to 20 carbon atoms; R^{2b}, R^{2c} and R^{2d} are each an aliphatic group having 1 to 20 carbon atoms or a partially or completely fluorinated aliphatic group having 1 to 20 carbon atoms, with the proviso that at least one of R^{2a} to R^{2d} is a partially or completely fluorinated aliphatic group having 1 to 20 carbon atoms; and m is a number of 1 to 20.

The viscosity of the fluorine-containing oil is e.g. at least 5 mPa·s, particularly 10 to 10,000 mPa·s. The viscosity of the fluorine oil (and the HFE) was measured by a Brookfield type viscometer or a capillary tube viscometer.

Specific examples of the perfluoropolyether include:
F(CF(CF₃)CF₂O)ₙCF₂CF₃ (Krytox manufactured by Du Pont Co.);
CF₃O(CF(CF₃)CF₂O)ₙ(CF₂O)ₘCF₃ (Fomblin Y manufactured by Montefluos Ltd.);
CF₃O(CF₂CF₂O)ₙ(CF₂O)ₘCF₃ (Fomblin Z manufactured by Montefluos Ltd.); and
F(CF₂CF₂CF₂O)ₙCF₂CF₃ (Demnum manufactured by Daikin Industries, Ltd.).

A number average molecular weight of the perfluoropolyether (as determined by ¹⁹F-NMR) is preferably in the range of 1,000 to 10,000.

In the general formulas (2) and (3), if R^{1a}, R^{1b}, R^{1c}, R^{1d}, R^{2a}, R^{2b}, R^{2c} and R^{2d} are partially or fully fluorinated aliphatic groups, these may contain an oxygen atom or an unsaturated bond. The partially fluorinated aliphatic group may be e.g. R¹⁰OCH₂ group (R¹⁰ is a partially or fully fluorinated aliphatic group (e.g. alkyl group)). Examples of the partially or fully fluorinated aliphatic group are as follows:
-CFH₂
-CF₂H
-CF₃
-CF₂CF₂H,
-CH₂CF₃,
-CF₂CFHCF₃,
-CF₂CH₃,
-CF₂CFH₂,
-C(CF₃)₂CH₃,
-CF₂CH(CF₃)₂,
-C(CF₃)₂CF₂H,
-C(CH₃)FCF₂CF₂CF₃,
-CH₂CF₂CH₂OH 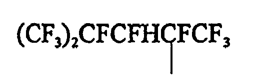 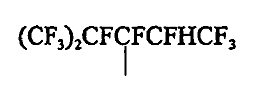 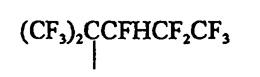 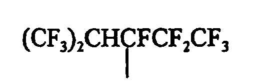 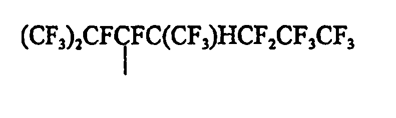 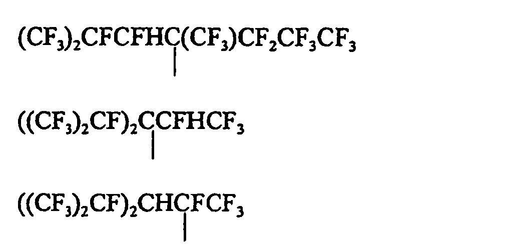
-CH₂CF₂COOR¹¹ (R¹¹ is a C₁ to C₁₀ aliphatic group).

If R^{1b}, R^{1c}, R^{2b}, R^{2c} and R^{2d} are a C₁ to C₂₀ aliphatic group, these may have an oxygen atom. Examples of the C₁ to C₂₀ aliphatic group include an alkyl group such as methyl, ethyl, n-propyl, isopropyl, n-butyl, isobutyl, sec-butyl and t-butyl as well as a hydroxyalkyl group such as hydroxymethyl and 2-hydroxyethyl (-CH₂CH₂OH).

R^{1b}, R^{1c}, R^{2b} and R^{2c} in the repeating unit may be the same or different.

The number of carbon atoms in each of R^{1a} to R^{1d} and R^{2a} to R^{2d} is from 1 to 10, particularly from 2 to 4.

Specific examples of the fluorine-based oil represented by the general formula (2) are as follows:

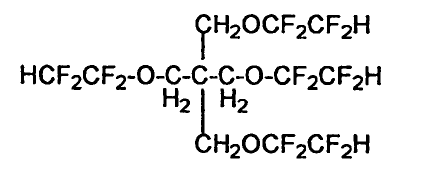

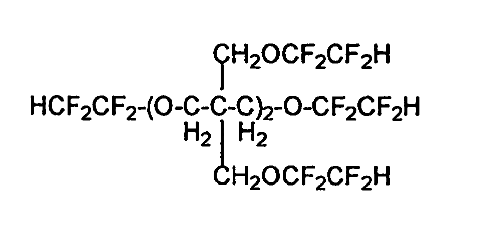

Specific examples of the fluorine-based oil represented by the general formula (3) are as follows:

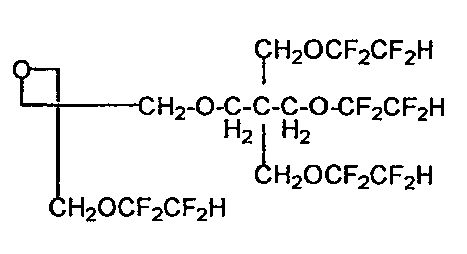

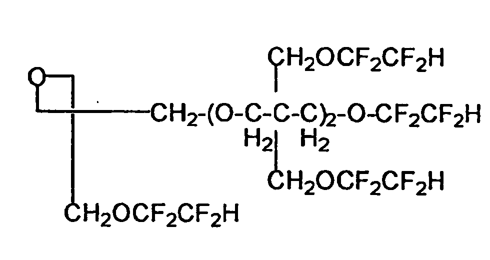

The fluorine-containing oil may be a mixture of at least two types. For example, a mixture consisting of the fluorine-based oils of the general formulas (2) and (3) at a weight ratio of from 5:95 to 95:5, for example 20:80 to 80:20, may be used as the fluorine-containing oil.

The HFE of the present invention is advantageously superior in the ability to disperse a fluorine compound-treated powder. By use of this advantage, the cosmetic having the characteristics never achieved in the past can be produced as well.

It is preferable that at least 1% by weight, based on the cosmetic, of the fluorine compound-treated powder and at least 5% by weight, based on the cosmetic, of the HFE are contained. For example, the amount of the fluorine compound-treated powder in the cosmetic is preferably 1 to 90% by weight, particularly 5 to 50% by weight, for example 5 to 20% by weight, based on the cosmetic. For example, the weight ratio of the fluorine compound-treated powder to the HFE may be in the range of from 1:100 to 1:5, preferably 1:90 to 1:10.

The fluorine compound-treated powder may be a powder which is surface-treated with a fluorine-containing phosphate ester represented, for example, by the general formula (4):

[Rf-A-O]ₙPO(OM)₃₋ₙ (4)

wherein Rf represents a C₆ to C₁₆ polyfluoroalkyl group or perfluoropolyether group,
"A" represents a C₁ to C₄ alkylene group, 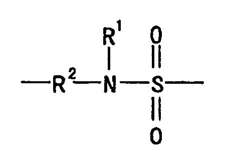 (wherein R¹ is a C₁ to C₄ alkyl group and R² is a C₁ to C₄ alkylene group), or 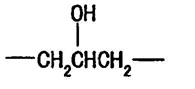 wherein "M" represents a hydrogen atom, a metal atom, ammonium or substituted ammonium, and n is a number of 1 to 3.

A base powder to be treated includes inorganic powder such as talc, kaolin, mica, mica titanium, titanium oxide, iron oxide, magnesium oxide, zinc monoxide, zinc dioxide, heavy or light calcium carbonate, dibasic calcium phosphate, aluminum hydroxide, barium sulfate, silica, alumina, silica gel, carbon black, antimony oxide, magnesium silicate aluminate and magnesium metasilicate aluminate, as well as an organic powder such as protein powder, fish scale, metallic soap, polyvinyl chloride, nylon-12, microcrystalline fiber powder and tar pigment.

The base powder may be treated with 1 to 10% by weight, relative to the base powder, of the fluorine-containing phosphate ester. The surface treatment may be generally surface coating.

In the present invention, suitable chemicals for modifying the feeling in use may be also employed in the step of surface treatment. Examples of the chemicals for modifying the feeling of the cosmetic powder in use include lecithin, N-mono long-chain acyl basic amino acids, silicone, chitosan, collagen and wax.

When the fluorine compound-treated powder is incorporated into the cosmetic, at least two types of the powders may be mixed.

Furthermore, the cosmetic may simultaneously comprise three ingredients, that is, HFE, the fluorine-containing oil and the fluorine compound-treated powder. Based on the cosmetic comprising three ingredients, the HFE may be contained in an amount of 5 to 99% by weight, preferably 10 to 90% by weight, the fluorine-containing oil may be contained in an amount of 0.5 to 90% by weight, preferably 5 to 50% by weight, and the fluorine compound-treated powder may be contained in an amount of 0.5 to 90% by weight, preferably 5 to 50% by weight.

The cosmetic of the present invention may contain solid or semi-solid oils such as Vaseline, lanolin, ceresin, microcrystalline wax, carnauba wax, candelilla wax, higher aliphatic acids and higher alcohols; liquid oils such as squalane, liquid paraffin, ester oils, diglycerides, triglycerides and silicone oil; fluorine-containing oils such as perfluoropolyether, perfluorodecalin and perfluoroctane; water- and oil-soluble polymers, surface active agents, coloring agents such as organic dyestuffs, ethanol, preservatives, antioxidants, pigments, thickening agents, pH adjusters, perfumes, UV absorbers, humectants, blood stream promoters, coolants, sweat regulators, germicides, skin activators etc.

### PREFERRED EMBODIMENT OF THE INVENTION

The examples of the present invention is described specifically, but this description does not limit the present invention.

### Example 1

50% by weight of a perfluoropolyether (Fomblin HC/04 manufactured by Audimont) (viscosity at 25°C: 70 mPa·s) was mixed with 50% by weight of C₄F₉OCH₃ (HFE A) (viscosity at 25°C: 0.6 mPa·s) under stirring to give a solution. Thereafter, a very small amount of a perfume was added to give a non-aqueous lotion. This non-aqueous lotion was felt very smooth in use. After the lotion was applied onto the skin, the HFE A evaporated rapidly and a uniform coating of the perfluoropolyether maintained the skin moisture.

### Example 2

A non-aqueous lotion was obtained in the same manner as in Example 1 except that the perfluoropolyether was replaced by a mixture (A/B = 8/2 by weight) of the fluorine-containing oil (viscosity at 25°C: 35 mPa·s) represented by chemical formula A: 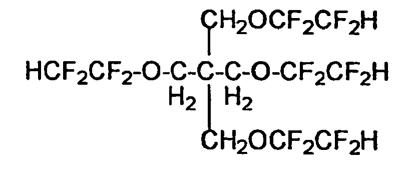 and the fluorine-containing oil (viscosity at 25°C: 40 mPa·s) represented by chemical formula B: 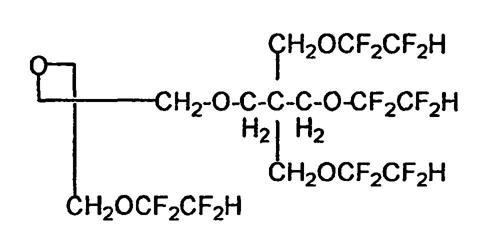 This non-aqueous location had the same properties as in Example 1.

### Example 3

A non-aqueous lotion was obtained in the same manner as in Example 1 except that the perfluoropolyether was replaced by a mixture (C/D = 7/3 by weight) of the fluorine-containing oil (viscosity at 25°C: 350 mPa·s) represented by chemical formula C: 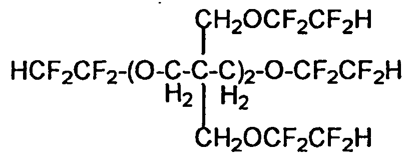 and the fluorine-containing oil (viscosity at 25°C: 400 mPa·s) represented by chemical formula D: 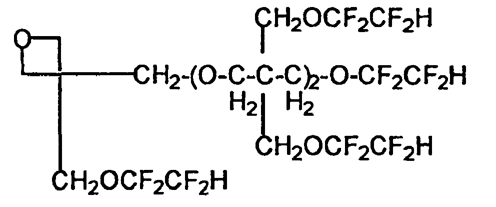 This non-aqueous location had the same properties as in Example 1.

In the Examples below, the mixture powder (a mixture of fluorine compound-treated powders) shown in Table 1 was used to produce a cosmetic. The fluorine compound-treated powder was prepared by treating a powder with 5% by weight of perfluoroalkyl ethyl phosphate ester diethanol amine salt ((C₉F₁₉CH₂CH₂O)ₙP(=O) [ONH₂(CH₂CH₂OH)₂]₃₋ₙ (average ofn = 1.8)).

**Table 1. Formulation of the Mixture Powder**

| Starting Materials | % by weight |
|---|---|
| (1) fluorine compound-treated titanium oxide | 8.0 |
| (2) fluorine compound-treated yellow iron oxide | 0.9 |
| (3) fluorine compound-treated red iron oxide | 0.3 |
| (4) fluorine compound-treated black iron oxide | 0.3 |
| (5) fluorine compound-treated talc | 36.1 |
| (6) fluorine compound-treated sericite | 50.6 |
| (7) fluorine compound-treated mica | 3.8 |

### Example 4

The powder ingredients in the formulation shown in Table 2 were mixed and ground with an atomizer and then transferred to a Henschel mixer. Then Fomblin HC/04 was added thereto, and the mixture was uniformly mixed. The mixture was dispersed in C₄H₉OC₂H₅ (HFE B) (viscosity at 25°C: 0.7 mPa·s) to give a solvent dispersion-type foundation. This solvent dispersion-type foundation was felt very smooth in use. After this foundation was applied onto the skin, the HFE B evaporated rapidly, and the resulting uniform coating maintained the skin moisture.

**Table 2. Non-Aqueous Dispersion-Type Foundation**

| Starting Materials | Blending Amount (% by weight) |
|---|---|
| (1) mixture powder (Table 1) | 10 |
| (2) perfluoropolyether (Fomblin HC/04) | 10 |
| (3) HFE B (C₄F₉OC₂H₅) | 80 |

### Example 5

The ingredients (1) to (8) in the formulation shown in Table 3 were mixed and ground in a colloid mill. After this mixture was heated at 80°C, the ingredients (9) to (13) which had been heated and mixed at 80°C were added thereto and emulsified uniformly in a homomixer. The ingredient (14) was added to this emulsion, further emulsified uniformly in a homomixer, and cooled to room temperature to give a liquid foundation. This liquid foundation was felt very smooth in use.

**Table 3. Liquid Foundation**

| Starting Materials | Blending Amount (% by weight) |
|---|---|
| (1) 1,3-butylene glycol | 10.00 |
| (2) carboxymethyl cellulose | 0.20 |
| (3) aluminum magnesium silicate | 0.20 |
| (4) mixture powder (Table 1) | 14.00 |
| (5) purified water | 50.75 |
| (6) sodium N-stearoyl-L-glutamate | 0.75 |
| (7) potassium hydroxide | 0.40 |
| (8) p-oxybenzoate | 0.20 |
| (9) glyceryl trioctanoate | 5.00 |
| (10) diisostearyl malate | 3.00 |
| (11) stearic acid | 3.00 |
| (12) glyceride monostearate | 1.50 |
| (13) cethanol | 1.00 |
| (14) HFE D (C₄F₉OC₄H₉) | 10.00 |

### Example 6

The fluorine compound-treated zinc oxide as the ingredient (3) shown in Table 4 was a product prepared by treating zinc oxide with 7% by weight, based on zinc oxide, of a perfluoroalkyl ethyl phosphate ester diethanolamine salt (the same as used in Example 3).

In the formulation shown in Table 4, the ingredients (1) and (2) were mixed and dissolved at room temperature, and the ingredient (3) was added thereto and dispersed with a disper. The ingredients (4) to (9) were added thereto under stirring and emulsified with a homomixer to give a sunscreen emulsion. This sunscreen emulsion was felt very smooth in use. After this emulsion was applied onto the skin, the HFE A evaporated rapidly, and the resulting uniform coating maintained the skin moisture.

**Table 4. Sunscreen Emulsion**

| Starting Materials | Blending Amount (% by weight) |
|---|---|
| (1) HFE A (C₄F₉OCH₃) | 40 |
| (2) fluorine-containing oil (the same as used in Example 2) | 10 |
| (3) fluorine compound-treated zinc oxide | 5 |
| (4) dimethylpolysiloxane-polyoxyalkylene copolymer | 3 |
| (5) glycerin | 2 |
| (6) ethanol | 5 |
| (7) water | 32.9 |
| (8) octyl methoxycinnamate | 2 |
| (9) perfume | 0.1 |

### Example 7

A set lotion was produced using the formulation shown in Table 5. After the ingredients (4) to (7) were dissolved, the ingredients (1) to (3) were added, moistened, and dissolved. Further, the ingredient (8) was added thereto slowly under stirring to give a set lotion. This set lotion was felt very smooth in use.

**Table 5. Set Lotion**

| Starting Materials | Blending Amount (% by weight) |
|---|---|
| (1) polyvinyl pyrrolidone | 5 |
| (2) polyoxyethylene (20) oleylether | 5 |
| (3) 1,3-butylene glycol | 10 |
| (4) HFE B (C₄F₉OC₂H₅) | 5 |
| (5) p-oxybenzoate ester | 0.1 |
| (6) colorant | Trace amount |
| (7) perfume | 0.5 |
| (8) purified water | Adjusted to 100 |

### EFFECT OF THE INVENTION

The HFE causing no environmental disruption and being highly safe for the skin is incorporated into the cosmetic, thereby increasing the solubility in the fluorine-containing oil and dispersibility in the fluorine compound-treated powder. The feeling of the cosmetic in use can be improved and the functionality of the cosmetic can be increased.

## Claims (Claims for the following Contracting State(s): FR, GB, IT)

1. A cosmetic comprising at least 1% by weight of a hydrofluoroether having a viscosity of less than 5 mPa·s at 25°C, represented by the general formula (1):
CₙF₂ₙ₊₁-O-Cₓ H₂ₓ₊₁ (1)
wherein n is a number of 1 to 6, and x is a number of 1 to 6, with the proviso that the cosmetic does not comprise a copolymer comprising
(A) 5 to 95% by weight of repeating units derived from a fluorine-containing (meth)acrylate, and
(B) 95 to 5 % by weight of repeating units derived from at least one fluorine-free monomer selected from the group consisting of a silicone macromonomer, a polyalkylene glycol (meth)acrylate, an alkyl (meth)acrylate macromonomer, and an alkyl (meth)acrylate,
wherein the copolymer comprises:
(i) a fluorine-containing (meth)acrylate and a silicone macromonomer,
(ii) a fluorine-containing (meth)acrylate and a polyalkylene glycol (meth)acrylate,
(iii) a fluorine-containing (meth)acrylate and an alkyl (meth)acrylate macromonomer,
(iv) a fluorine-containing (meth)acrylate and a C₁₋₄ alkyl-containing alkyl (meth)acrylate,
(v) a fluorine-containing (meth)acrylate, a silicone macromonomer, and a C₁₋₂₂ alkyl-containing alkyl (meth)acrylate, or
(vi) a fluorine-containing (meth)acrylate, a silicone macromonomer, and an alkyl (meth)acrylate macromonomer.

2. The cosmetic according to claim 1, which comprises at least 1% by weight of a fluorine-containing oil having a viscosity of at least 5 mPa·s at 25°C and at least 5% by weight of the hydrofluoroether.

3. The cosmetic according to claim 2, wherein the fluorine-containing oil may be a perfluoropolyether, a fluorine-based oil represented by the general formula (2): 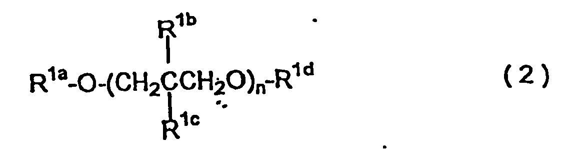 wherein R^{1a} and R^{1d} are each a hydrogen atom or a partially or completely fluorinated aliphatic group having 1 to 20 carbon atoms; R^{1b} and R^{1c} are each a hydrogen atom, or an aliphatic group having 1 to 20 carbon atoms, or a partially or completely fluorinated aliphatic group having 1 to 20 carbon atoms, with the proviso that at least one of R^{1a} to R^{1d} is a partially or completely fluorinated aliphatic group having 1 to 20 carbon atoms; and n is a number of 1 to 20, or a fluorine-based oil represented by the general formula (3): 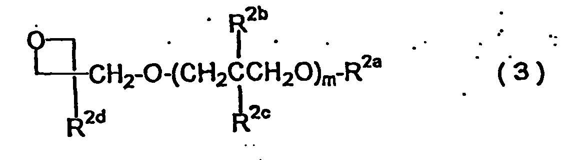 wherein R^{2a} is a hydrogen atom or a partially or completely fluorinated aliphatic group having 1 to 20 carbon atoms; R^{2b}, R^{2c} and R^{2d} are each an aliphatic group having 1 to 20 carbon atoms or a partially or completely fluorinated aliphatic group having 1 to 20 carbon atoms, with the proviso that at least one of R^{2a} to R^{2d} is a partially or completely fluorinated aliphatic group haying 1 to 20 carbon atoms; and m is a number of 1 to 20.

4. The cosmetic according to claim 1, which comprises at least 1% by weight of a fluorine compound-treated powder and at least 5% by weight of the hydrofluoroether.

5. The cosmetic according to claim 4, wherein the fluorine compound-treated powder is a powder surface-treated with a fluorine-containing phosphate ester represented by the general formula (4):
[Rf-A-O]ₙPO(OM)₃₋ₙ (4)
wherein Rf represents a C₆ to C₁₆ polyfluoroalkyl group or perfluoropolyether group, A represents a C₁ to C₄ alkylene group, 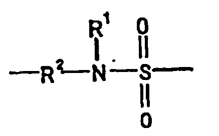 (wherein R¹ is a C₁ to C₄ alkyl group and R² is a C₁ to C₄ alkylene group), or 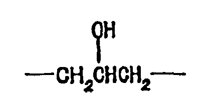 wherein M represents a hydrogen atom, a metal atom, ammonium or substituted ammonium, and n is a number of 1 to 3.

6. Use of a hydrofluoroether having a viscosity of less than 5 mPa·s at 25°C, represented by the general formula (1):
CₙF₂ₙ₊₁-O-CₓH₂ₓ₊₁ (1)
wherein n is a number of 1 to 6, and x is a number of 1 to 6, as solvent in a cosmetic with the proviso that the cosmetic does not comprise the copolymer as defined in claim 1.

7. The use of claim 6 wherein the cosmetic is a finishing cosmetic such as foundation, face powder, cheek color and eye color, a fundamental cosmetic such as face lotion, milky lotion, cream and sunscreen milky lotion, a hair-caring product such as rinse and conditioner or a lipstick overcoat.

## Claims (Claims for the following Contracting State(s): AT, BE, CH, CY, DE, DK, ES, FI, FR, GB, GR, IE, IT, LI, LU, MC, NL, PT, SE)

1. A cosmetic comprising at least 1% by weight of a hydrofluoroether having a viscosity of less than 5 mPa·s at 25°C, represented by the general formula (1):
CₙF₂ₙ₊₁-O-CₓH₂ₓ₊₁ (1)
wherein n is a number of 1 to 6, and x is a number of 1 to 6.

2. The cosmetic according to claim 1, which comprise at least 1% by weight of a fluorine-containing oil having a viscosity of at least 5 mPa·s at 25°C and at least 5% by weight of the hydrofluoroether.

3. The cosmetic according to claim 2, wherein the fluorine-containing oil may be a perfluoropolyether,
a fluorine-based oil represented by the general formula (2): 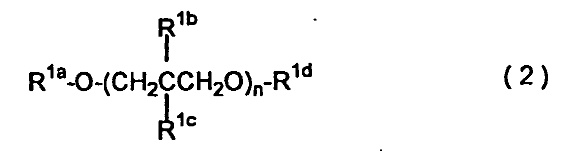 wherein R^{1a} and R^{1d} are each a hydrogen atom or a partially or completely fluorinated aliphatic group having 1 to 20 carbon atoms; R^{1b} and R^{1c} are each a hydrogen atom, or an aliphatic group having 1 to 20 carbon atoms, or a partially or completely fluorinated aliphatic group having 1 to 20 carbon atoms, with the proviso that at least one of R^{1a} to R^{1d} is a partially or completely fluorinated aliphatic group having 1 to 20 carbon atoms; and n is a number of 1 to 20, or
a fluorine-based oil represented by the general formula (3): 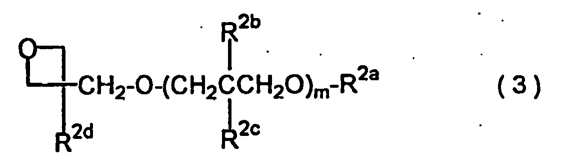 wherein R^{2a} is a hydrogen atom or a partially or completely fluorinated aliphatic group having 1 to 20 carbon atoms; R^{2b}, R^{2c} and R^{2d} are each an aliphatic group having 1 to 20 carbon atoms or a partially or completely fluorinated aliphatic group having 1 to 20 carbon atoms, with the proviso that at least one of R^{2a} to R^{2d} is a partially or completely fluorinated aliphatic group having 1 to 20 carbon atoms; and m is a number of 1 to 20.

4. The cosmetic according to claim 1, which comprises at least 1% by weight of a fluorine compound-treated powder and at least 5% by weight of the hydrofluoroether.

5. The cosmetic according to claim 4, wherein the fluorine compound-treated powder is a powder surface-treated with a fluorine-containing phosphate ester represented by the general formula (4):
[Rf-A-O]ₙPO(OM)₃₋ₙ (4)
wherein Rf represents a C₆ to C₁₆ polyfluoroalkyl group or perfluoropolyether group,
A represents a C₁ to C₄ alkylene group, 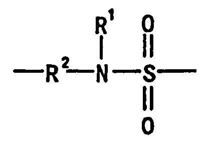 (wherein R¹ is a C₁ to C₄ alkyl group and R² is a C₁ to C₄ alkylene group), or 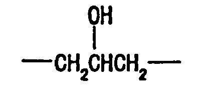 wherein M represents a hydrogen atom, a metal atom, ammonium or substituted ammonium, and n is a number of 1 to 3

6. Use of a hydrofluoroether having a viscosity of less than 5 mPa.s at 25°C, represented by the general formula (1):
CₙF₂ₙ₊₁-O-CₓH₂ₓ₊₁ (1)
wherein n is a number of 1 to 6, and x is a number of 1 to 6 as solvent in a cosmetic.

7. The use of claim 6, wherein the cosmetic is a finishing cosmetic such as foundation, face powder, cheek colour and eye colour, a fundamental cosmetic, such as face lotion, milky lotion, cream and sunscreen milky lotion, a hair-caring product, such as rinse and conditioner or a lipstick overcoat.

## Patentansprüche (Patentansprüche für folgende(n) Vertragsstaat(en): AT, BE, CH, CY, DE, DK, ES, FI, FR, GB, GR, IE, IT, LI, LU, MC, NL, PT, SE)

1. Kosmetikum, umfassend mindestens 1 Gew.% eines Hydrofluoroethers mit einer Viskosität von weniger als 5 mPa·s bei 25°C, repräsentiert durch die allgemeine Formel (1):
CₙF₂ₙ₊₁-O-CₓH₂ₓ₊₁ (1)
worin n eine Zahl von 1 bis 6 ist und x ist eine Zahl von 1 bis 6.

2. Kosmetikum gemäß Anspruch 1, das mindestens 1 Gew.% eines fluorhaltiges Öls mit einer Viskosität von mindestens 5 mPa·s bei 25°C und mindestens 5 Gew.% des Hydrofluoroethers umfaßt.

3. Kosmetikum gemäß Anspruch 2, wobei das fluorhaltige Öl ein Perfluorpolyether, ein auf Fluor basierendes Öl, repräsentiert durch die allgemeine Formel (2): 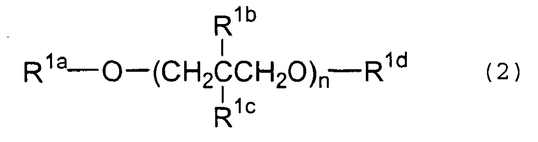 worin R^{1a} und R^{1d} jeweils ein Wasserstoffatom oder eine teilweise oder vollständig fluorierte aliphatische Gruppe mit 1 bis 20 Kohlenstoffatomen sind; R^{1b} und R^{1c} sind jeweils ein Wasserstoffatom oder eine aliphatische Gruppe mit 1 bis 20 Kohlenstoffatomen oder eine teilweise oder vollständig fluorierte aliphatische Gruppe mit 1 bis 20 Kohlenstoffatomen, mit der Maßgabe, daß mindestens eines von R^{1a} bis R^{1d} eine teilweise oder vollständig fluorierte aliphatische Gruppe mit 1 bis 20 Kohlenstoffatomen ist; und n ist eine ganze Zahl von 1 bis 20,
oder ein auf Fluor basierendes Öl, repräsentiert durch die allgemeine Formel (3): 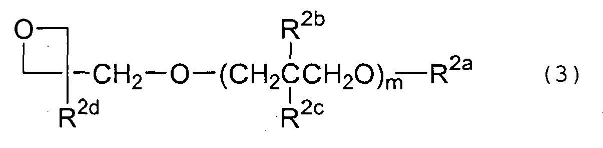 worin R^{2a} ein Wasserstoffatom oder eine teilweise oder vollständig fluorierte aliphatische Gruppe mit 1 bis 20 Kohlenstoffatomen ist; R^{2b}, R^{2c} und R^{2d} sind jeweils eine aliphatische Gruppe mit 1 bis 20 Kohlenstoffatomen oder eine teilweise oder vollständig fluorierte aliphatische Gruppe mit 1 bis 20 Kohlenstoffatomen, mit der Maßgabe, daß mindestens eines von R^{2a} bis R^{2d} eine teilweise oder vollständig fluorierte aliphatische Gruppe mit 1 bis 20 Kohlenstoffatomen ist; und m ist eine Zahl von 1 bis 20, sein kann.

4. Kosmetikum gemäß Anspruch 1, das mindestens 1 Gew.% eines Fluorverbindungs-behandelten Pulvers und mindestens 5 Gew.% des Hydrofluoroethers umfaßt.

5. Kosmetikum gemäß Anspruch 4, wobei das Fluorverbindungsbehandelte Pulver ein Pulver ist, das mit einem fluorhaltigen Phosphatester oberflächenbehandelt wurde, der durch die allgemeine Formel (4) repräsentiert wird:
[Rf-A-O]ₙPO(OM)₃₋ₙ (4)
worin Rf eine C₆₋₁₆-Polyfluoralkylgruppe oder Perfluorpolyethergruppe repräsentiert, A repräsentiert eine C₁₋₄-Alkylengruppe, 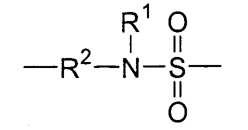 (worin R¹ eine C₁₋₄-Alkylgruppe und R² eine C₁₋₄-Alkylengruppe ist), oder 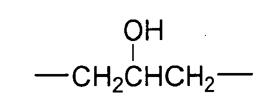 worin M ein Wasserstoffatom, ein Metallatom, Ammonium oder substituiertes Ammonium repräsentiert, und n ist eine Zahl von 1 bis 3.

6. Verwendung eines Hydrofluoroethers mit einer Viskosität von weniger als 5 mPa·s bei 25°C, repräsentiert durch die allgemeine Formel (1):
CₙF₂ₙ₊₁-O-CₓH₂ₓ₊₁ (1)
worin n eine Zahl von 1 bis 6 ist und x ist eine Zahl von 1 bis 6, als Lösungsmittel in einem Kosmetikum.

7. Verwendung gemäß Anspruch 6, wobei das Kosmetikum ein Finishing-Kosmetikum ist, wie z.B. eine Grundierung, Gesichtspuder, Rouge und Lidschatten, ein Grundkosmetikum wie z.B. eine Gesichtslotion, milchige Lotion, Creme und Sonnenschutzmilchlotion, ein Haarpflegeprodukt wie z.B. eine Spülung und ein Conditioner oder eine Lippenstift-Überschicht.

## Patentansprüche (Patentansprüche für folgende(n) Vertragsstaat(en): FR, GB, IT)

1. Kosmetikum, umfassend mindestens 1 Gew.% eines Hydrofluoroethers mit einer Viskosität von weniger als 5 mPa·s bei 25°C, repräsentiert durch die allgemeine Formel (1):
CₙF₂ₙ₊₁-O-CₓH₂ₓ₊₁ (1)
worin n eine Zahl von 1 bis 6 ist und x ist eine Zahl von 1 bis 6, mit der Maßgabe, daß das Kosmetikum kein Copolymer umfaßt, umfassend:
(A) 5 bis 95 Gew.% von sich wiederholenden Einheiten, abgeleitet von einem fluorhaltigen (Meth)acrylat und
(B) 95 bis 5 Gew.% von sich wiederholenden Einheiten, abgeleitet von mindestens einem fluorfreien Monomer, ausgewählt aus der Gruppe bestehend aus einem Silicon-Makromonomer, einem Polyalkylenglycol(meth)acrylat, einem Alkyl(meth)acrylat-Makromonomer und einem Alkyl(meth)acrylat,
wobei das Copolymer folgendes umfaßt:
(i) ein fluorhaltiges (Meth)acrylat und ein Silicon-Makromonomer,
(ii) ein fluorhaltiges (Meth)acrylat und ein Polyalkylenglycol(meth)acrylat,
(iii) ein fluorhaltiges (Meth)acrylat und ein Alkyl(meth)acrylat-Makromonomer,
(iv) ein fluorhaltiges (Meth)acrylat und ein C₁₋₄-alkylhaltiges Alkyl(meth)acrylat,
(v) ein fluorhaltiges (Meth)acrylat, ein Silicon-Makromonomer und ein C₁₋₂₂-alkylhaltiges Alkyl(meth)acrylat oder
(vi) ein fluorhaltiges (Meth)acrylat, ein Silicon-Makromonomer und ein Alkyl(meth)acrylat-Makromonomer.

2. Kosmetikum gemäß Anspruch 1, das mindestens 1 Gew.% eines fluorhaltiges Öls mit einer Viskosität von mindestens 5 mPa·s bei 25°C und mindestens 5 Gew.% des Hydrofluoroethers umfaßt.

3. Kosmetikum gemäß Anspruch 2, wobei das fluorhaltige Öl ein Perfluorpolyether, ein auf Fluor basierendes Öl, repräsentiert durch die allgemeine Formel (2): 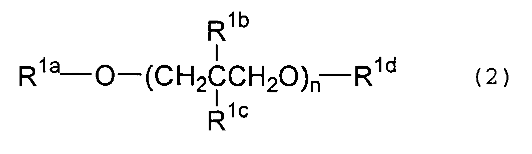 worin R^{1a} und R^{1d} jeweils ein Wasserstoffatom oder eine teilweise oder vollständig fluorierte aliphatische Gruppe mit 1 bis 20 Kohlenstoffatomen sind; R^{1b} und R^{1c} sind jeweils ein Wasserstoffatom oder eine aliphatische Gruppe mit 1 bis 20 Kohlenstoffatomen oder eine teilweise oder vollständig fluorierte aliphatische Gruppe mit 1 bis 20 Kohlenstoffatomen, mit der Maßgabe, daß mindestens eines von R^{1a} bis R^{1d} eine teilweise oder vollständig fluorierte aliphatische Gruppe mit 1 bis 20 Kohlenstoffatomen ist; und n ist eine ganze Zahl von 1 bis 20,
oder ein auf Fluor basierendes Öl, repräsentiert durch die allgemeine Formel (3): 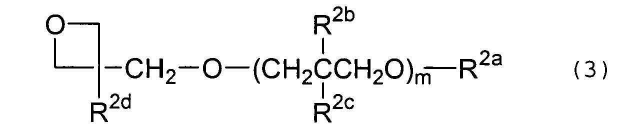 worin R^{2a} ein Wasserstoffatom oder eine teilweise oder vollständig fluorierte aliphatische Gruppe mit 1 bis 20 Kohlenstoffatomen ist; R^{2b}, R^{2c} und R^{2d} sind jeweils eine aliphatische Gruppe mit 1 bis 20 Kohlenstoffatomen oder eine teilweise oder vollständig fluorierte aliphatische Gruppe mit 1 bis 20 Kohlenstoffatomen, mit der Maßgabe, daß mindestens eines von R^{2a} bis R^{2d} eine teilweise oder vollständig fluorierte aliphatische Gruppe mit 1 bis 20 Kohlenstoffatomen ist; und m ist eine Zahl von 1 bis 20, sein kann.

4. Kosmetikum gemäß Anspruch 1, das mindestens 1 Gew.% eines Fluorverbindungs-behandelten Pulvers und mindestens 5 Gew.% des Hydrofluoroethers umfaßt.

5. Kosmetikum gemäß Anspruch 4, wobei das Fluorverbindungsbehandelte Pulver ein Pulver ist, das mit einem fluorhaltigen Phosphatester oberflächenbehandelt wurde, der durch die allgemeine Formel (4) repräsentiert wird:
[Rf-A-O]ₙPO(OM)₃₋ₙ (4)
worin Rf eine C₆₋₁₆-Polyfluoralkylgruppe oder Perfluorpolyethergruppe repräsentiert, A repräsentiert eine C₁₋₄-Alkylengruppe, 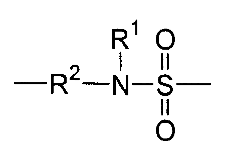 (worin R¹ eine C₁₋₄-Alkylgruppe und R² eine C₁₋₄-Alkylengruppe ist), oder 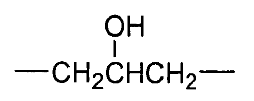 worin M ein Wasserstoffatom, ein Metallatom, Ammonium oder substituiertes Ammonium repräsentiert, und n ist eine Zahl von 1 bis 3.

6. Verwendung eines Hydrofluoroethers mit einer Viskosität von weniger als 5 mPa·s bei 25°C, repräsentiert durch die allgemeine Formel (1):
CₙF₂ₙ₊₁-O-Cₓ H₂ₓ₊₁ (1)
worin n eine Zahl von 1 bis 6 ist und x ist eine Zahl von 1 bis 6, als Lösungsmittel in einem Kosmetikum, mit der Maßgabe, daß das Kosmetikum nicht das Copolymer umfaßt wie definiert in Anspruch 1.

7. Verwendung gemäß Anspruch 6, wobei das Kosmetikum ein Finishing-Kosmetikum ist, wie z.B. eine Grundierung, Gesichtspuder, Rouge und Lidschatten, ein Grundkosmetikum wie z.B. eine Gesichtslotion, milchige Lotion, Creme und Sonnenschutzmilchlotion, ein Haarpflegeprodukt wie z.B. eine Spülung und ein Conditioner oder eine Lippenstift-Überschicht.

## Revendications (Revendications pour l'(les) Etat(s) contractant(s) suivant(s): FR, GB, IT)

1. Cosmétique comprenant au moins 1 % en masse d'un hydrofluoroéther ayant une viscosité inférieure à 5 mPa.s à 25°C, représenté par la formule générale (1) :
CₙF₂ₙ₊₁-O-Cₓ H₂ₓ₊₁ (1)
où n est un nombre de 1 à 6, et x est un nombre de 1 à 6, avec la condition que le cosmétique ne comprend pas un copolymère comprenant
(A) 5 à 95 % en masse d'unités répétées dérivées d'un (méth)acrylate contenant du fluor, et
(B) 95 à 5 % en masse d'unités répétées dérivées d'au moins un monomère sans fluor choisi dans le groupe consistant en un macromonomère de silicone, un (méth)acrylate de polyalkylèneglycol, un macromonomère de (méth)acrylate d'alkyle, et un (méth)acrylate d'alkyle,
où le copolymère comprend :
(i) un (méth)acrylate contenant du fluor et un macromonomère de silicone,
(ii) un (méth)acrylate contenant du fluor et un (méth)acrylate de polyalkylèneglycol,
(iii) un (méth)acrylate contenant du fluor et un macromonomère de (méth)acrylate d'alkyle,
(iv) un (méth)acrylate contenant du fluor et un (méth)acrylate d'alkyle contenant un C₁₋₄ alkyle,
(v) un (méth)acrylate contenant du fluor, un macromonomère de silicone et un (méth)acrylate d'alkyle contenant un C₁₋₂₂ alkyle, ou
(vi) un (méth)acrylate contenant du fluor, un macromonomère de silicone et un macromonomère de (méth)acrylate d'alkyle.

2. Cosmétique selon la revendication 1 qui comprend au moins 1 % en masse d'une huile contenant du fluor ayant une viscosité d'au moins 5 mPa.s à 25°C et au moins 5 % en masse de l'hydrofluoroéther.

3. Cosmétique selon la revendication 2 où l'huile contenant du fluor peut être un perfluoropolyéther, une huile à base de fluor représentée par la formule générale (2) : 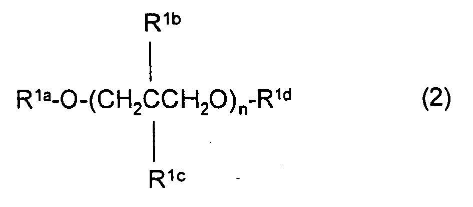 où R^{1a} et R^{1d} sont chacun un atome d'hydrogène ou un groupe aliphatique partiellement ou complètement fluoré ayant 1 à 20 atomes de carbone ; R^{1b} et R^{1c} sont chacun un atome d'hydrogène, un groupe aliphatique ayant 1 à 20 atomes de carbone ou un groupe aliphatique partiellement ou complètement fluoré ayant 1 à 20 atomes de carbone, avec la condition qu'au moins l'un de R^{1a} à R^{1d} est un groupe aliphatique partiellement ou complètement fluoré ayant 1 à 20 atomes de carbone ; et n est un nombre de 1 à 20, ou une huile à base de fluor représentée par la formule générale (3) : 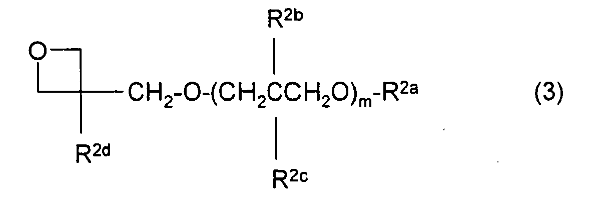 où R^{2a} est un atome d'hydrogène ou un groupe aliphatique partiellement ou complètement fluoré ayant 1 à 20 atomes de carbone ; R^{2b}, R^{2c} et R^{2d} sont chacun un groupe aliphatique ayant 1 à 20 atomes de carbone ou un groupe aliphatique partiellement ou complètement fluoré ayant 1 à 20 atomes de carbone, avec la condition qu'au moins l'un de R^{2a} à R^{2d} est un groupe aliphatique partiellement ou complètement fluoré ayant 1 à 20 atomes de carbone ; et m est un nombre de 1 à 20.

4. Cosmétique selon la revendication 1 qui comprend au moins 1 % en masse d'une poudre traitée avec un composé du fluor et au moins 5 % en masse de l'hydrofluoroéther.

5. Cosmétique selon la revendication 4 où la poudre traitée avec un composé du fluor est une poudre traitée en surface avec un ester phosphate contenant du fluor représenté par la formule générale (4) :
[Rf-A-O]ₙPO(OM)₃₋ₙ (4)
où Rf représente un groupe C₆ à C₁₆ polyfluoroalkyle ou un groupe perfluoropolyéther, A représente un groupe C₁ à C₄ alkylène, 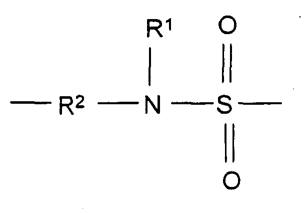 (où R¹ est un groupe C₁ à C₄ alkyle et R² est un groupe C₁ à C₄ alkylène), ou 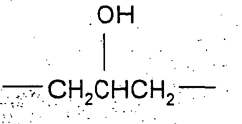 où M représente un atome d'hydrogène, un atome métallique, un ammonium ou ammonium substitué, et n est un nombre de 1 à 3.

6. Utilisation d'un hydrofluoroéther ayant une viscosité inférieure à 5 mPa.s à 25°C représenté par la formule générale (1) :
CₙF₂ₙ₊₁-O-CₓH₂ₓ₊₁ (1)
où n est un nombre de 1 à 6, et x est un nombre de 1 à 6, comme solvant dans un cosmétique avec la condition que le cosmétique ne comprend pas le copolymère tel que défini dans la revendication 1.

7. Utilisation selon la revendication 6 où le cosmétique est un cosmétique de finition comme un fond de teint, une poudre pour le visage, une couleur pour les joues et une couleur pour les yeux, un cosmétique fondamental comme une lotion pour le visage, une lotion laiteuse, une crème et une lotion laiteuse de type écran solaire, un produit pour les soins des cheveux comme un produit de rinçage et un conditionneur ou un revêtement de rouge à lèvres.

## Revendications (Revendications pour l'(les) Etat(s) contractant(s) suivant(s): AT, BE, CH, CY, DE, DK, ES, FI, FR, GB, GR, IE, IT, LI, LU, MC, NL, PT, SE)

1. Composition cosmétique comprenant au moins 1 % en masse d'un hydrofluoroéther ayant une viscosité inférieure à 5 mPa.s à 25°C, représentée par la formule générale (1) :
CₙF₂ₙ₊₁-O-Cₓ H₂ₓ₊₁ (1)
où n est un nombre de 1 à 6 et x est un nombre de 1 à 6.

2. Cosmétique selon la revendication 1 qui comprend au moins 1 % en masse d'une huile contenant du fluor ayant une viscosité d'au moins 5 mPa.s à 25°C et au moins 5 % en masse de l'hydrofluoroéther.

3. Cosmétique selon la revendication 2 où l'huile contenant du fluor peut être
un perfluoropolyéther,
une huile à base de fluor représentée par la formule générale (2) : 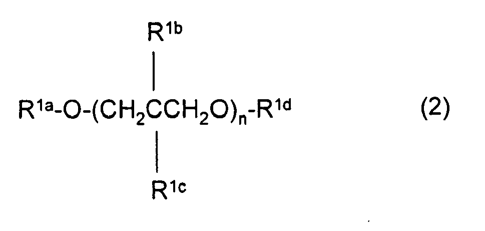 où R^{1a} et R^{1d} sont chacun un atome d'hydrogène ou un groupe aliphatique partiellement ou complètement fluoré ayant 1 à 20 atomes de carbone ; R^{1b} et R^{1c} sont chacun un atome d'hydrogène, ou un groupe aliphatique ayant 1 à 20 atomes de carbone ou un groupe aliphatique partiellement ou complètement fluoré ayant 1 à 20 atomes de carbone, avec la condition qu'au moins l'un de R^{1a} à R^{1d} est un groupe aliphatique partiellement ou complètement fluoré ayant 1 à 20 atomes de carbone ; et n est un nombre de 1 à 20, ou
une huile à base de fluor représentée par la formule générale (3) : 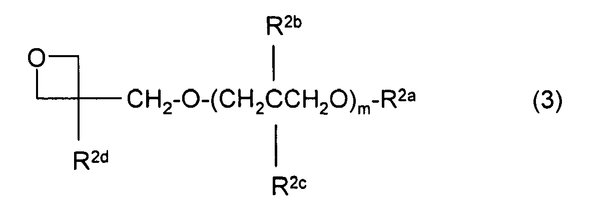 où R^{2a} est un atome d'hydrogène ou un groupe aliphatique partiellement ou complètement fluoré ayant 1 à 20 atomes de carbone ; R^{2b}, R^{2c} et R^{2d} sont chacun un groupe aliphatique ayant 1 à 20 atomes de carbone ou un groupe aliphatique partiellement ou complètement fluoré ayant 1 à 20 atomes de carbone, avec la condition qu'au moins l'un de R^{2a} à R^{2d} est un groupe aliphatique partiellement ou complètement fluoré ayant 1 à 20 atomes de carbone ; et m est un nombre de 1 à 20.

4. Cosmétique selon la revendication 1 qui comprend au moins 1 % en masse d'une poudre traitée avec un composé du fluor et au moins 5 % en masse de l'hydrofluoroéther.

5. Cosmétique selon la revendication 4 où la poudre traitée avec un composé du fluor est une poudre traitée en surface avec un ester phosphate contenant du fluor représenté par la formule générale (4) :
[Rf-A-O]ₙPO(OM)₃₋ₙ (4)
où Rf représente un groupe C₆ à C₁₆ polyfluoroalkyle ou un groupe perfluoropolyéther,
A représente un groupe C₁ à C₄ alkylène, 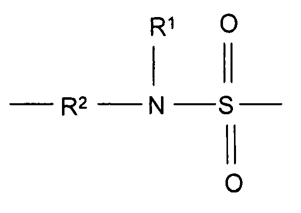 (où R¹ est un groupe C₁ à C₄ alkyle et R² est un groupe C₁ à C₄ alkylène), ou 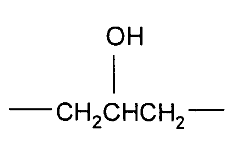 où M représente un atome d'hydrogène, un atome métallique, un ammonium ou ammonium substitué, et n est un nombre de 1 à 3.

6. Utilisation d'un hydrofluoroéther ayant une viscosité inférieure à 5 mPa.s à 25°C, représenté par la formule générale (1) :
CₙF₂ₙ₊₁-O-CₓH₂ₓ₊₁ (1)
où n est un nombre de 1 à 6, et x est un nombre de 1 à 6, comme solvant dans un cosmétique.

7. Utilisation selon la revendication 6 où le cosmétique est un cosmétique de finition comme un fond de teint, une poudre pour le visage, une couleur pour les joues et une couleur pour les yeux, un cosmétique fondamental, comme une lotion pour le visage, une lotion laiteuse, une crème et une lotion laiteuse de type écran solaire, un produit pour les soins des cheveux, comme un produit de rinçage et un conditionneur ou un revêtement de rouge à lèvres.
